# DEMANDE DE BREVET EUROPEEN

(11) **EP 0 738 510 A2**
(43) Date de publication de la demande: **23.10.1996**
(21) Numéro de dépôt: 96400697.7
(22) Date de dépôt: 29.03.1996
(51) Int. Cl.: A61K 7/48

(54) **Utilisation d'un inhibiteur d'hmg coenzyme A reductase pour lutter contre le vieillissement de la peau**

(30) Priorité: 20.04.1995 FR 9504747
(71) Demandeur: L'OREAL, F-75008 Paris (FR)
(72) Inventeur: Breton, Lionel, 78000 Versailles (FR); de Lacharriere, Olivier, 75015 Paris (FR)
(74) Mandataire: Lhoste, Catherine

(57) **Abrégé**

L'invention se rapporte à l'utilisation d'au moins un inhibiteur d'HMG-Coenzyme A-réductase comme actif antivieillissement dans une composition cosmétique et/ou dermatologique. Elle a aussi pour objet l'utilisation d'au moins un inhibiteur d'HMG-Coenzyme A-réductase pour lutter contre les rides et/ou les ridules et/ou les taches de la peau humaine et/ou pour raviver l'éclat de la peau et/ou pour traiter l'acné.

L'invention se rapporte aussi à une composition cosmétique à usage topique contenant au moins un inhibiteur d'HMG-Coenzyme A-réductase et au moins un actif possédant des propriétés desquamantes, agissant en synergie.

## Description

L'invention se rapporte à l'utilisation d'un inhibiteur d'HMG-Coenzyme A-réductase comme actif antivieillissement dans ou pour la fabrication d'une composition cosmétique et/ou dermatologique. Elle a aussi pour objet l'utilisation d'un inhibiteur d'HMG-Coenzyme A-réductase pour lutter contre les rides et/ou les ridules et/ou les taches de la peau humaine et/ou raviver l'éclat de la peau et/ou pour traiter l'acné. Elle se rapporte aussi à une composition à usage topique contenant un inhibiteur d'HMG-Coenzyme A-réductase et un actif possédant des propriétés desquamantes, agissant en synergie.

Le vieillissement cutané résulte des effets sur la peau de facteurs intrinsèques et extrinsèques. Cliniquement, les signes du vieillissement se traduisent par l'apparition de rides et ridules, par un relâchement des tissus cutanés et sous-cutanés, par une perte de l'élasticité cutanée, par une atonie de la texture de la peau, et par le jaunissement de la peau qui devient plus terne et sans éclat. Sur les zones de la peau qui ont été exposées au soleil tout au long de la vie - essentiellement le visage, le décolleté, les mains et les avant bras - on observe souvent des taches pigmentaires, des télangiectasies et une élastose.

Certains de ces signes sont plus particulièrement liés au vieillissement intrinsèque ou physiologique, c'est-à-dire au vieillissement lié à l'âge, alors que d'autres sont plus spécifiques du vieillissement extrinsèque, c'est-à-dire du vieillissement provoqué d'une manière générale par l'environnement ; il s'agit plus particulièrement du photo-vieillissement dû à l'exposition au soleil, à la lumière ou à tout autre rayonnement.

Depuis plusieurs années, sont apparues sur le marché des compositions cosmétiques contenant des α-hydroxy-acides, tels que les acides glycolique, malique et lactique, pour lutter contre les signes visibles du vieillissement, notamment contre les rides, les ridules, le teint terne et les taches, ainsi que pour faire disparaître les comédons dus à l'acné. Ces compositions se présentent généralement sous forme de crèmes ou de lotions.

Pour être actives, ces compositions doivent contenir l'α-hydroxy-acide sous sa forme acide (voir notamment l'article de W.P. Smith, Soap Cosmetic Chemical Specialities, Septembre 1993, pp. 54-58 et 76 : "Hydroxy acids and skin aging"), ce qui confère à la composition un pH bas, entraînant des problèmes de tolérance cutanée qui se traduisent, pour l'utilisateur, par des picotements, des rougeurs et des tiraillements pouvant conduire à un inconfort important.

Par ailleurs, l'acné est essentiellement la conséquence de deux phénomènes intriqués : la formation du comédon et l'inflammation péricomédonienne ou folliculite, la formation du comédon étant elle-même la conséquence de deux phénomènes : l'obstruction du canal pilo-sébacé et la production accrue de sébum par les glandes sébacées, donnant lieu à la formation de points noirs. L'acné n'est pas inflammatoire au stade de la formation du comédon, mais le devient par suite de la prolifération de germes résultant de la rétention séborrhéique et de l'hyperproduction de sébum. Les germes en cause sont notamment des germes anaérobies diphthéroïdes tels que les *Propionibacteria* *(acnés, granulosum, avidum).* Au stade de la formation du comédon, le traitement peut consister simplement à épurer la peau, tandis qu'au stade d'inflammation, il convient d'effectuer un traitement anti-inflammatoire.

Pour traiter l'acné, on utilise généralement des agents kératolytiques tels que les rétinoïdes, et notamment l'acide rétinoïque, des anti-inflammatoires tels que les peroxydes, et notamment le peroxyde de benzoyle, et des agents antiséborrhéiques. Ces actifs présentent l'inconvénient d'être assez irritants, et cet effet irritant est d'autant plus ressenti par le sujet traité que celui-ci a la peau sensible.

La demanderesse a donc cherché des actifs ayant le même effet que les α-hydroxy-acides ou que les agents kératolytiques sans poser ces problèmes d'intolérance.

Elle a trouvé de manière inattendue que les inhibiteurs d'HMG-Coenzyme A-réductase, permettaient d'atteindre ce but.

Les inhibiteurs d'HMG-Coenzyme A-réductase (β-hydroxy-β-méthylClutaryl-Coenzyme A-réductase notée ci-après HMG-CoA-réductase) sont des actifs pharmaceutiques utilisés par voie orale dans le traitement des hypercholestérolémies (taux plasmatiques de cholestérol trop élevés). Voir à cet effet le document « Pharmacologie, Des concepts fondamentaux aux applications thérapeutiques », publié sous la direction de M. Schorderet, Editions Frison-Roche, seconde édition, 1992.

Ces inhibiteurs sont également utilisés dans le traitement des maladies liées à la présence excessive de cholestérol, telles que l'artériosclérose.

L'HMG-Co A-réductase est une enzyme qui intervient très précocement dans la synthèse du cholestérol. Au niveau épidermique, il est classiquement reconnu que le cholestérol ainsi que ses métabolites jouent un rôle primordial dans la cohésion des cellules épidermiques et tout particulièrement des cornéocytes (cellules constitutives du *stratum comeum).*

Le document EP-A-369263 décrit l'utilisation des inhibiteurs d'HMG-CoA-réductase pour le traitement topique des maladies de la peau, notamment des kératoses et du psoriasis. Par ailleurs, E. Proksch et al. décrivent que l'application topique d'inhibiteur d'HMG-CoA-réductase perturbe la fonction barrière de la peau (voir British Journal of dermatology, 1993, vol.128, n°5, p. 473-482), et M. Krasovec et al. indiquent que les rougeurs eczémateuses de certains patients traités par un inhibiteur d'HMG-CoA-réductase peuvent être dues à un disfonctionnement de la fonction barrière (voir Dermatology, 1993, vol. 186, n°4, p. 248-252).

Toutefois, aucun document ne décrit ni ne suggère l'utilisation des inhibiteurs d'HMG-CoA-réductase comme actif permettant d'atténuer, voire supprimer, les signes du vieillissement cutané. Et aucun document ne décrit ni ne suggère l'utilisation des inhibiteurs d'HMG-CoA-réductase pour traiter l'acné.

Aussi, la présente invention a pour objet l'utilisation d'au moins un inhibiteur d'HMG-Coenzyme A-réductase comme actif antivieillissement dans et/ou pour la fabrication d'une composition cosmétique et/ou dermatologique.

De façon plus précise, l'invention a encore pour objet l'utilisation d'au moins un inhibiteur d'HMG-Coenzyme A-réductase dans une composition cosmétique pour lutter contre les rides et/ou les ridules et/ou les taches actiniques et/ou les dyschromies cutanées de la peau humaine et/ou raviver l'éclat de la peau humaine.

L'invention a également pour objet l'utilisation d'au moins un inhibiteur d'HMG-Coenzyme A-réductase pour la fabrication d'une composition dermatologique pour lutter contre les rides et/ou les taches actiniques et/ou les dyschromies et/ou les cicatrices et/ou l'ichtyose de la peau humaine et/ou l'acné.

Les inhibiteurs d'HMG-CoA-réductase peuvent être par exemple les composés suivants : mévastatine, lovastatine, pravastatine, simvastatine, fluvastatine, dalvastatine, et leurs dérivés et sels, notamment leurs sels de sodium.

Dans les compositions selon l'invention, l'inhibiteur d'HMG CoA-réductase (ou un mélange d'inhibiteurs) est utilisé en une quantité allant de 0,000001 à 2 % en poids par rapport au poids total de la composition et en particulier en une quantité allant de 0,00001 à 0,1 % en poids par rapport au poids total de la composition.

En outre, la demanderesse a observé que ces composés peuvent être associés à d'autres actifs, connus pour leur propriété desquamante, par exemple les hydroxy-acides, les α- ou β-céto-acides ou les rétinoïdes. Elle a constaté de manière surprenante qu'une telle association permet de diminuer la concentration active de ces produits du fait de l'addition de leurs effets. On peut ainsi obtenir une composition moins irritante, et moins toxique ainsi qu'une composition plus efficace.

Ainsi, l'invention a aussi pour objet une composition cosmétique contenant, dans un milieu cosmétiquement acceptable, au moins un inhibiteur d'HMG-Coenzyme A-réductase et au moins un actif possédant des propriétés desquamantes, agissant en synergie.

Les hydroxyacides peuvent être par exemple des α-hydroxyacides ou des β-hydroxyacides, qui peuvent être linéaires, ramifiés ou cycliques, saturés ou insaturés. Les atomes d'hydrogène de la chaîne carbonée peuvent, en outre, être substitués par des halogènes, des radicaux halogénés, alkylés, acylés, acyloxylés, alcoxy carbonylés ou alcoxylés ayant de 2 à 18 atomes de carbone.

Les hydroxyacides qui peuvent être utilisés sont notamment les acides glycolique, lactique, malique, tartrique, citrique et de manière générale les acides de fruits, les acides hydroxy-2 alcanoïque, mandélique, salicylique, ainsi que leurs dérivés alkylés comme l'acide n-octanoyl-5-salicylique, l'acide n-dodécanoyl-5-salicylique, l'acide n-décanoyl-5-salicylique, l'acide n-octyl-5-salicylique, l'acide n-heptyloxy-5 ou -4-salicylique, l'acide 2-hydroxy-3-méthyl-benzoïque ou encore leurs dérivés alcoxylés comme l'acide 2-hydroxy-3-méthoxybenzoïque. Ces hydroxy-acides peuvent se trouver sous forme libre ou salifiée, en particulier sous forme de sels obtenus par salification avec une base minérale ou organique.

Les rétinoïdes peuvent être notamment l'acide rétinoïque (all-trans ou 13-cis) et ses dérivés, le rétinol (vitamine A) et ses esters tels que le palmitate de rétinol, l'acétate de rétinol et le propionate de rétinol ainsi que leurs sels.

A titre d'exemple, les hydroxyacides, les céto-acides et les rétinoïdes peuvent être utilisés dans les compositions selon l'invention en une quantité représentant de 0,1 à 10 %, de préférence de 0,1 à 5 % et mieux de 0,5 à 3 % en poids du poids total de la composition.

La demanderesse a constaté que la desquamation de la peau consistant à éliminer les cellules superficielles de la peau entraînait un lissage des traits, un ravivage du teint, une réduction des rides et ridules ainsi qu'une diminution voire une élimination des taches actiniques et/ou des dyschromies de la peau. Aussi, la demanderesse a déterminé l'efficacité du traitement des signes du vieillissement des inhibiteurs d'HMG-Coenzyme A-réductase en effectuant un test *in vitro* de desquamation.

Ce test *in vitro* a été réalisé sur kératinocytes en utilisant de l'acide n-octanoyl-5-salicylique, de la mévastatine et un mélange des deux. Le principe du test repose sur le fait que la desquamation induit la libération de cornéocytes. Le pouvoir de desquamation du produit testé va être d'autant plus grand que le nombre de cornéocytes libérés sera important.

Le protocole du test a été le suivant : à partir de biopsies de peau, on a obtenu, par séparation de l'épiderme, les kératinocytes que l'on a dissociés par action enzymatique à la trypsine et mis en culture à la concentration de 2.10⁻⁵ cellules/ml. La croissance et la différenciation des kératinocytes a été obtenue par culture durant 10 à 20 jours en milieu spécifique.

Puis, après élimination du milieu de culture, on a ajouté le produit à tester et évalué l'activité du produit. Pour ce faire, on a réalisé deux prélèvements à T0 et T60, c'est-à-dire avant l'ajout du produit et 60 minutes après cet ajout, et on a analysé les prélèvements ainsi effectués au cytomètre de flux pour dénombrer la population de cornéocytes. Au cytomètre de flux, les populations de cornéocytes et de kératinocytes sont différenciées par traitement à l'acridine orange spécifique de l'ADN des cellules, qui se lie au noyau des cellules et révèle donc exclusivement la présence des kératinocytes.

L'indice de détachement cellulaire est déterminé par la différence entre T60 et T0.

La même mesure a été réalisée pour un témoin ne contenant pas de produit à tester car l'expérience produit inévitablement la libération de cornéocytes, même en absence d'actif. La variation du témoin a fixé arbitrairement la norme de 100 %.

Les résultats sont rassemblés dans le tableau ci-dessous :

| Témoin | Mévastatine (Composé I) 5.10⁻⁷M | Acide n-octanoyl-5 salicylique (Composé II) 5.10⁻⁵M | (Composé I à 5.10⁻⁷M) + (Composé II à 5.10⁻⁵M) |
|---|---|---|---|
| 100 % | 299,5 % | 219 % | 393 % |

Ces résultats montrent que la mévastatine, à une concentration 100 fois plus faible que l'acide n-octanoyl-5-salicylique, est beaucoup plus active que celui-ci, et que le mélange des deux ne conduit pas à une saturation de l'effet de desquamation, mais au contraire à une addition des effets de chacun de ces composés. Cet effet additif montre que les processus de desquamation de la mévastatine et de l'acide n-octanoyl-5 salicylique sont différents. ll en résulte un effet additif de l'actif desquamant (acide n-octanoyl-5-salicylique) et de l'inhibiteur d'HMG-Coenzyme A-réductase.

L'invention a encore pour objet un procédé de traitement cosmétique et/ou dermatologique pour traiter les rides et/ou ridules de la peau humaine et/ou pour raviver l'éclat de la peau humaine et/ou pour traiter l'acné, consistant à appliquer sur la peau une composition contenant au moins un inhibiteur d'HMG-Coenzyme A-réductase.

La composition de l'invention contient un milieu cosmétiquement ou dermatologiquement acceptable, c'est-à-dire un milieu compatible avec la peau, y compris le cuir chevelu, les muqueuses et les tissus humains. La composition contenant l'inhibiteur d'HMG-CoA-réductase peut être appliquée par voie topique sur le visage, le cou, les muqueuses ou toute autre zone cutanée du corps humain.

Les compositions selon l'invention peuvent se présenter sous toutes les formes appropriées pour une application topique, notamment sous forme de solutions aqueuses, hydroalcooliques ou huileuses, de dispersions du type lotion ou sérum, de gels aqueux, anhydres ou huileux, d'émulsions obtenues par dispersion d'une phase grasse dans une phase aqueuse (H/E) ou inversement (E/H), de microémulsions, ou encore de microcapsules, de microparticules, ou de dispersions de vésicules lipidiques de type ionique et/ou non ionique. Ces compositions sont préparées selon les méthodes usuelles.

Ces compositions peuvent se présenter éventuellement sous forme de compositions pour aérosol contenant également un agent propulseur sous pression.

Les quantités des différents constituants des compositions selon l'invention sont celles classiquement utilisées dans les domaines considérés.

Ces compositions constituent notamment des crèmes, laits, lotions, gels ou mousses de protection, de traitement ou de soin du visage, des mains, du corps et/ou des muqueuses, ou de nettoyage de la peau.

Les compositions peuvent également consister en des préparations solides constituant des savons ou des pains de nettoyage.

Lorsque la composition de l'invention est une émulsion, la proportion de la phase grasse peut aller de 5 % à 80 % en poids, et de préférence de 5 % à 50 % en poids par rapport au poids total de la composition. Les huiles, les émulsionnants et les coémulsionnants utilisés dans la composition sous forme d'émulsion sont choisis parmi ceux classiquement utilisés dans le domaine cosmétique ou dermatologique. L'émulsionnant et le coémulsionnant sont présents, dans la composition, en une proportion allant de 0,3 % à 30 % en poids, et de préférence de 0,5 à 20 % en poids par rapport au poids total de la composition. L'émulsion peut, en outre, contenir des vésicules lipidiques.

Lorsque la composition est une solution ou un gel huileux, la quantité d'huile peut aller jusqu'à plus de 90 % en poids du poids total de la composition.

De façon connue, la composition de l'invention peut contenir également des adjuvants habituels dans les domaines cosmétique et dermatologique, tels que les gélifiants hydrophiles ou lipophiles, les actifs hydrophiles ou lipophiles, les conservateurs, les antioxydants, les solvants, les parfums, les charges, les filtres, les absorbeurs d'odeur et les matières colorantes. Les quantités de ces différents adjuvants sont celles classiquement utilisées dans les domaines considérés, et par exemple de 0,01 % à 20 % du poids total de la composition. Ces adjuvants, selon leur nature, peuvent être introduits dans la phase grasse, dans la phase aqueuse et/ou dans les vésicules lipidiques.

Comme huiles utilisables dans l'invention, on peut citer les huiles minérales (huile de vaseline), les huiles végétales (huile de karité, huile d'amande douce), les huiles animales, les huiles de synthèse, les huiles siliconées (cyclométhicone) et les huiles fluorées (perfluoropolyéthers). On peut aussi utiliser comme matières grasses des alcools gras, des acides gras (acide stéarique), des cires (paraffine, carnauba, cire d'abeilles).

Comme émulsionnants utilisables dans l'invention, on peut citer le Polysorbate 60 et le stéarate de sorbitan vendus respectivement sous les dénominations commerciales Tween 60 et Span 60 par la Société ICI. On peut y ajouter des coémulsionnants tels que le PPG-3 myristyl éther vendu sous la dénomination commerciale Emcol 249-3K par la société Witco.

Comme solvants utilisables dans l'invention, on peut citer les alcools inférieurs comme l'éthanol et l'isopropanol, et les glycols tels que le propylène glycol.

Comme gélifiants hydrophiles, on peut citer les polymères carboxyvinyliques (carbomer), les copolymères acryliques tels que les copolymères d'acrylates/alkylacrylates, les poly(méth)acrylates de glycéryle tels que le produit vendu sous la dénomination de Norgel par la société Guardian, les polyacrylamides et notamment le mélange de polyacrylamide, C13-14-Isoparaffine et Laureth-7, vendu sous la dénomination de Sepigel 305 par la société Seppic, les polysaccharides tels que l'hydroxypropylcellulose, les gommes naturelles (xanthane) et les argiles, et, comme gélifiants lipophiles, on peut citer les argiles modifiées comme les bentones, les sels métalliques d'acides gras comme les stéarates d'aluminium, la silice hydrophobe, les polyéthylènes et l'éthylcellulose.

Comme actifs hydrophiles, on peut utiliser les protéines ou les hydrolysats de protéine, les acides aminés, les polyols, l'urée, l'allantoïne, les sucres et les dérivés de sucre, les vitamines hydrosolubles, l'amidon, les extraits bactériens ou végétaux, notamment d'Aloe Vera.

Comme actifs lipophiles, on peut utiliser le tocophérol (vitamine E) et ses dérivés, les acides gras essentiels, les céramides, les huiles essentielles.

On peut, entre autre, associer les inhibiteurs d'HMG-CoA-réductase à des agents actifs destinés notamment à la prévention et/ou au traitement des affections cutanées. Parmi ces agents actifs, on peut citer à titre d'exemple :
- les agents modulant la différenciation et/ou la prolifération et/ou la pigmentation cutanée tels que la vitamine D et ses dérivés, les estrogènes tels que l'estradiol, l'acide kojique ou l'hydroquinone ;
- les antibactériens tels que le phosphate de clindamycine, l'érythromycine ou les antibiotiques de la classe des tétracyclines ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox ;
- les agents anti-inflammatoires stéroïdiens, tels que l'hydrocortisone, les anthralines (dioxyanthranol), les anthranoïdes, le valérate de bétaméthasone ou le propionate de clobétasol ;
- les agents anti-radicaux libres, tels que l'alpha-tocophérol ou ses esters, les superoxyde dismutases, certains chélatants de métaux ou l'acide ascorbique et ses esters ;
- les antiséborrhéiques tels que la progestérone ;
- les antiacnéiques comme l'acide rétinoïque ou le peroxyde de benzoyle ;
- les antiseptiques.

Le procédé de traitement de l'invention peut être mis en oeuvre notamment en appliquant les compositions hygiéniques, cosmétiques ou dermatologiques telles que définies ci-dessus, selon la technique d'utilisation habituelle de ces compositions. Par exemple : application de crèmes, de gels, de sérums, de lotions, de laits sur la peau, le cuir chevelu et/ou les muqueuses humains.

Les exemples suivants illustrent l'invention. Dans ces exemples, les proportions indiquées sont des pourcentages en poids.

### Exemple 1: Emulsion H/E

### Phase A :

- Acide n-octanoyl-5-salicylique 0,5
- Mévastatine 0,001
- Huile d'amande douce 14,5
- Huile de karité 7,0
- PPG-3 myristyl éther (EMCOL 249-3K) 5,0
- Conservateur (propylparabène) 0,1
- Polysorbate 60 (TWEEN 60) 2,5
- Stéarate de sorbitan (SPAN 60) 2,5

### Phase B :

- Cyclométhicone 4,0
- Gomme de xanthane 0,2
- Polymère carboxyvinylique 0,5

### Phase C :

- Triéthanolamine (neutralisant) 0,5
- Eau 2,0

### Phase D :

- Conservateur (méthylparabène) 0,2
- Glycérine 5,0
- Eau qsp 100

### Mode opératoire :

On fait fondre les constituants de la phase A à 85° C, puis on refroidit la phase A à 70° C et on y introduit les phases B puis C et D sous agitation. On refroidit jusqu'à la température ambiante.

On obtient une crème de jour qui, après plusieurs jours d'application, confère à la peau un aspect plus lisse et plus jeune qu'avant le traitement et diminue les taches.

### Exemple 2 : Gel

Fluvastatine (sel de sodium) 0,005
Hydroxypropylcellulose (Klucel H vendu par la société Hercules) 1,0
Antioxydant 0,05
Isopropanol 40,0
Conservateur 0,3
Eau qsp 100

On obtient un gel qui, en application régulière, permet d'estomper les taches de la peau.

### Exemple 3 : Solution pour application dermatologique

Mévastatine 0,05
Antioxydant 0,05
Alcool éthylique 10
Conservateur 0,3
Eau qsp 100

L'application sous contrôle dermatologique de cette solution permet d'obtenir un effacement des taches et dyschromies, un estompement des rides et ridules et une amélioration de l'état clinique de la peau, dont l'aspect devient celui d'une peau plus jeune.

Cette application se fait à raison d'une à trois séances hebdomadaires pendant 4 à 6 semaines.

### Exemple 4 : Gel pour traiter l'acné

- Polyacrylate de glycéryle (Norgel) 29,5 %
- Polyacrylamide/C13-14 lsoparaffine/laureth-7 (Sepigel 305) 2 %
- Huile de silicone 10 %
- Lovastatine 0,05 %
- Sel de sodium de l'EDTA (séquestrant) 0,1 %
- Conservateur 0,4 %
- Eau qsp 100 %

Le gel obtenu en application quotidienne convient pour le traitement de l'acné.

## Revendications

1. Utilisation d'au moins un inhibiteur d'HMG-Coenzyme A-réductase comme actif antivieillissement dans et/ou pour la fabrication d'une composition cosmétique et/ou dermatologique.

2. Utilisation d'au moins un inhibiteur d'HMG-Coenzyme A-réductase dans une composition cosmétique pour lutter contre les rides et/ou les ridules et/ou les taches actiniques et/ou les dyschromies cutanées de la peau humaine et/ou raviver l'éclat de la peau.

3. Utilisation d'au moins un inhibiteur d'HMG-Coenzyme A-réductase pour la fabrication d'une composition dermatologique pour lutter contre les rides et/ou les taches actiniques et/ou les dyschromies cutanées et/ou les cicatrices et/ou l'ichtyose et/ou l'acné.

4. Utilisation selon l'une quelconque des revendications 1 à 3, caractérisée en ce que l'inhibiteur d'HMG-Coenzyme A-réductase est choisi dans le groupe comprenant la mévastatine, la lovastatine, la pravastatine, la simvastatine, la fluvastatine, la dalvastatine et leurs mélanges.

5. Utilisation selon l'une quelconque des revendications précédentes, caractérisée en ce que l'inhibiteur d'HMG CoA-réductase est utilisé en une quantité allant de 0,000001 à 2 % en poids par rapport au poids total de la composition.

6. Utilisation selon l'une quelconque des revendications précédentes, caractérisée en ce que la composition comprend, en outre, au moins un actif choisi parmi les α-hydroxy-acides, les β-hydroxy-acides, les α-céto-acides, les β-céto-acides, les rétinoïdes.

7. Utilisation selon l'une quelconque des revendications précédentes, caractérisée en ce que la composition comprend, en outre, au moins un actif choisi parmi les acides glycolique, lactique, malique, tartrique, citrique, hydroxy-2 alcanoïque, mandélique, salicylique, l'acide n-octanoyl-5-salicylique.

8. Utilisation selon la revendication 6 ou 7, caractérisée en ce que l'actif est présent en une quantité allant de 0,1 à 5 % en poids par rapport au poids total de la composition.

9. Utilisation selon l'une quelconque des revendications précédentes, caractérisée en ce que la composition contient, en outre, au moins un adjuvant choisi parmi les protéines ou les hydrolysats de protéine, les acides aminés, les polyols, l'urée, les sucres et les dérivés de sucre, les vitamines, l'amidon, les extraits végétaux, les acides gras essentiels, les céramides, les huiles essentielles.

10. Utilisation selon l'une quelconque des revendications précédentes, caractérisée en ce que la composition est une solution aqueuse, huileuse ou hydroalcoolique, une émulsion eau-dans-huile, une émulsion huile-dans-eau, une microémulsion, un gel aqueux, un gel anhydre, un sérum, une dispersion de vésicules, de microcapsules ou de microparticules.

11. Composition cosmétique contenant, dans un milieu cosmétiquement acceptable, au moins un inhibiteur d'HMG-Coenzyme A-réductase et au moins un actif possédant des propriétés desquamantes, agissant en synergie.

12. Composition selon la revendication 11, caractérisée en ce que l'inhibiteur d'HMG-Coenzyme A-réductase est choisi dans le groupe comprenant la mévastatine, la lovastatine, la pravastatine, la simvastatine, la fluvastatine, la dalvastatine et leurs mélanges.

13. Composition selon la revendication 11 ou 12, caractérisée en ce que l'actif est choisi parmi les α-hydroxy-acides, les β-hydroxy-acides, les α-céto-acides, les β-céto-acides, les rétinoïdes et leurs mélanges.

14. Composition selon l'une quelconque des revendications 11 à 13, caractérisée en ce que l'actif est choisi parmi les acides glycolique, lactique, malique, tartrique, citrique, hydroxy-2 alcanoïque, mandélique, salicylique, l'acide n-octanoyl-5-salicylique et leurs mélanges.

15. Composition selon l'une quelconque des revendications 11 à 14, caractérisée en ce que le milieu cosmétiquement acceptable est une solution aqueuse, huileuse ou hydroalcoolique, une émulsion eau-dans-huile, une émulsion huile-dans-eau, une microémulsion, un gel aqueux, un gel anhydre, un gel huileux, un sérum, une dispersion de vésicules.

16. Composition selon l'une quelconque des revendications 11 à 15, caractérisée en ce que l'inhibiteur d'HMG- Coenzyme A-réductase est utilisé en une quantité allant de 0,000001 à 2 % en poids par rapport au poids total de la composition.

17. Procédé cosmétique pour traiter les rides et/ou ridules de la peau humaine et/ou pour raviver l'éclat de la peau, consistant à appliquer sur la peau une composition contenant au moins un inhibiteur d'HMG-Coenzyme A-réductase.

18. Procédé cosmétique pour traiter l'acné, consistant à appliquer sur la peau une composition contenant au moins un inhibiteur d'HMG-Coenzyme A-réductase.
